# EUROPEAN PATENT APPLICATION

(11) **EP 3 290 111 A1**
(43) Date of publication of application: **07.03.2018**
(21) Application number: 16186599.3
(22) Date of filing: 31.08.2016
(51) Int. Cl.: B01J 20/286, B01J 20/32, B01D 15/38, C07K 14/62

(54) **SORBENT FOR THE SEPARATION OF INSULIN FROM A SOLUTION**

(71) Applicant: InstrAction GmbH, 68199 Mannheim (DE)
(72) Inventor: BECKER, Juliane, 68163 Mannheim (DE); MEYER, Christian, 68723 Schwetzingen (DE); WELTER, Martin, 69151 Neckargemünd (DE)
(74) Representative: RatnerPrestia

(57) **Abstract**

The invention relates to the use of a sorbent for the improved separation of insulin or derivatives of insulin from solutions, the sorbent itself as well as a method for preparing a sorbent.

## Description

The present invention relates to the use of a sorbent for the improved separation of insulin or derivatives of insulin from solutions, the sorbent itself as well as a method for preparing a sorbent.

Insulin is a hormone which is vitally important for diabetics, and is preferably administered subcutaneously. Therefore there is a high demand on a cost-efficient method for the production of high purity insulin. The fermentative production of insulin by bacteria or fungi has the advantage that no animal organism has to be used. However, the drawback is the separation of insulin-related by-products, such as A21-desamido-insulin. Since some of the by-products are distinguished from insulin by lipophilic groups and others by hydrophilic groups the separation or purification is generally only obtained by a two-stage or multi-stage method. Such methods are therefore time-consuming and cost-intensive.

In case ion exchangers are used for the separation of insulin from solutions, there is the disadvantage of a low selectivity vis-à-vis insulin-related by-products, in particular if the differences between insulin and the by-products are of lipophilic nature. Furthermore, ion exchangers are usually based on relatively large particles, which typically come along with only low pressure stability. Larger particles often involve a deterioration of the chromatographic resolution, a low linear flow rate and therefore a low productivity.

When using chromatographic phases based on lipophilized silica, such as silica-based C18-phases, for the separation of insulin there is usually the disadvantage of a lower stability against caustic sanitization reagents, such as sodium hydroxide. Furthermore, those phases do in general not exhibit good separation properties, in particular if the difference between insulin and the by-product is of ionic nature.

Therefore, it was the object of the present invention to provide the use of a sorbent or the sorbent itself, allowing to provide high purity insulin in excellent yields in a cost-efficient way and without significant effort.

The present invention solves this problem in that a sorbent for the separation of insulin or derivatives of insulin from solution is used or provided, wherein the sorbent comprises porous particles of a crosslinked sulfonated aromatic polymer, the porous particles being coated with a hydrophilic polymer, the hydrophilic polymer comprising a ligand group in its side chain, the ligand group being an aliphatic hydrocarbon unit with 3 to 20 carbon atoms. The latter one is the use (of the sorbent) according to the invention. The present invention is, however, also directed to the sorbent itself. The term "sorbent according to the invention" as herein used is referred to as also comprising the "use of the sorbent according to this invention".

In other words, the present invention is also directed to a method for the separation of insulin or derivatives of insulin from a solution comprising the step of bringing into contact the solution with a sorbent, wherein the sorbent comprises porous particles of a crosslinked sulfonated aromatic polymer, the particles being coated with a hydrophilic polymer, wherein the hydrophilic polymer comprises a ligand group in its side chain, the ligand group being an aliphatic hydrocarbon group having 3 to 20 carbon atoms. All preferred embodiments and definitions with respect to the use according to the inventions are also valid for the method according to the present invention.

According to the invention it is preferred that the insulin is human insulin. The insulin is preferably produced via fermentation. However, the use of the sorbent according to the invention is not limited to insulin produced via fermentation. By the method according to the invention insulin may also be separated from solutions which are obtained from animal bodies.

The term *"derivative of insulin"* as herein used is referred to as insulin-related proteins which are formed by the production of insulin via fermentation or via animal bodies. A prominent representative of these derivatives is A21-desamido-insulin.

The term *"separation"* as herein used is on the one hand referred to as the production/separation of the target molecule (insulin or a derivative of insulin) from solution as well as to the separation of undesired macromolecules from the solution in a way that the target molecule maintains in the purified solution.

The term *"the solution"* from which the insulin is to be separated is referred to as all solutions which comprise in addition to insulin and the solvent (preferably water) at least one further substance in dissolved or suspended form. Preferably the solution is a fermentation solution.

The skeletal structure of the crosslinked sulfonated aromatic polymer may be any polymeric skeletal structure known in the art, which comprises or consists of hydrocarbon-containing repeating units.

The term *"hydrocarbon-containing repeating units"* is referred to as any conceivable compound, which is mainly composed of carbon and hydrogen, but may also contain heteroatoms.

The linkage of repeating units to become a polymer may be affected by any polymerization method known in the art. According to the invention radical, cationic or anionic olefin polymerization is preferred.

The skeletal structure of the crosslinked sulfonated aromatic polymer is particularly preferred a polyvinyl structure. The skeletal structure is preferably a crosslinked skeletal structure thereby forming a crosslinked polymer. In particular in case of a polyvinyl structure the crosslinking is obtained via a copolymerization of a vinyl group-containing monomer with a monomer containing two or more vinyl groups. It is in principle conceivable that firstly a polymer is produced which has a linear skeletal structure; the subsequent crosslinking may then be obtained by the reaction of functional groups in the side chain with a crosslinking reagent.

The crosslinked sulfonated aromatic polymer preferably contains the aromatic unit in its side chain or alternatively in the crosslinking units.

Furthermore the crosslinked sulfonated aromatic polymer preferably comprises sulfonic acid groups in its side chain.

The side chain of the crosslinked sulfonated aromatic polymer is preferably a sulfonated aromatic unit as further described below. Preferably the sulfonated aromatic units are bound to the skeletal structure of the polymer via a covalent single bond. Further, the sulfonated aromatic units may be substituted with an aliphatic radical. It is further preferred that the sulfonated aromatic unit is directly bound to an atom of the skeletal structure via a covalent single bond.

The term *"aromatic unit"* in the present invention is referred to a mono- or polycyclic aromatic ring system which may be substituted. In the sense of the present invention the term *"aromatic ring system"* is preferably referred to as an aromatic ring system with 6 to 60 carbon atoms, preferably 6 to 30 carbon atoms and most preferred 6 to 10 carbon atoms. The aromatic ring system may be monocyclic or polycyclic, i.e. it may comprise one ring (for instance phenyl) or two or more rings, which may be condensed (for instance naphthyl) or may be covalently connected (for instance biphenyl), or may be a combination of condensed and connected rings.

Preferred aromatic ring systems are for example phenyl, biphenyl, triphenyl, naphthyl, anthracyl, binaphthyl, phenanthryl, dihydrophenanthryl, pyrene, dihydropyrene, chrysene, perylene, tetracene, pentacene, benzpyrene, fluorene and indene. More preferred is the aromatic ring system phenyl, biphenyl or naphthyl, most preferred phenyl.

As already mentioned above the aromatic ring system may be substituted by an aliphatic radical. It is thereby conceivable that the aromatic ring system is not only substituted by one, but may also be substituted by two or more aliphatic radicals.

An aliphatic radical is preferably a hydrocarbon radical having 1 to 20 or 1 to 10 carbon atoms, respectively. Aliphatic hydrocarbon radicals according to the invention are preferably linear, branched or cyclic alkyl groups, wherein one or more hydrogen atoms may be substituted by fluorine.

It is strongly preferred that the sulfonated aromatic unit substituted or unsubstituted with an aliphatic radical is a phenyl sulfonic acid group or a derivative thereof. Derivatives of the phenyl sulfonic acid group are preferably derivatives which are substituted with an aliphatic radical. In this case the sulfonic acid group is preferably in the para-position of the phenyl radical with respect to the position of the phenyl ring binding to the skeletal structure. The aliphatic radical is hereby preferably a methyl or ethyl group, which is in the ortho- and/or meta-position of the phenyl group with respect to the position of the phenyl group which binds to the skeletal structure. It is, however, in particular preferred that the sulfonated aromatic unit is not substituted. In this connection, in particular a sulfonated crosslinked polystyrene comes into consideration. The crosslinking of the sulfonated polystyrene preferably occurs by copolymerization of styrene with divinylbenzol, with a subsequent sulfonation of the phenyl groups. However, any other crosslinking reagent containing two or more vinyl groups are conceivable for the production of a crosslinked copolymer.

The crosslinking degree of the crosslinked sulfonated polymer is preferably in the range of 0.5 to 50 %, more preferred 5 to 45 % and mostly preferred 10 to 35 %. In the present invention the specification of the crosslinking degree in percentage is referred to as the mol-percentage of the used compound containing two vinyl groups with respect to the total amount of the monomer units to be polymerized.

The sulfonation degree of the crosslinked sulfonated polymer is preferably in the range of 1 to 80 %, more preferred in the range of 3 to 60 % and mostly preferred in the range of 5 to 40 %. The sulfonation degree in percentage is referred to the mol-number of sulfonic acid groups in relation to all monomer units which are used for the polymerization which comprise a sulfonatable group. In case sulfonated polystyrene-divinylbenzol-copolymer is used as the crosslinked sulfonated polymer, the sulfonation degree in percentage is referred to the number of sulfonic acid groups in relation to all phenyl- or phenylene-groups present in the polymer.

Since the porous particles are of an organic polymer there is the advantage of an excellent chemical stability against caustic sanitization agents in contrast to common carrier materials, such as silica. Thus, the sorbent according to the invention can be separated or purified in a simplified manner and can be re-used.

According to the present invention the crosslinked sulfonated aromatic polymer is present in the form of porous particles. The porous particles may have a regular or irregular form. The term *"regular form"* is herein referred to as a form which may be presented by symmetry operations, such as reflection, point reflection, rotation axis or combinations thereof. A preferred regular form is the spherical form. The term *"spherical"* is herein not only referred to as a mere symmetric sphere, but also forms slightly differing from spheres, such as an ellipse. This term should also comprise two combined spherical bodies formed to a bar-bell. The term *"irregular form"* is herein referred to as any form which does not comprise any symmetry.

The porous particles preferably comprise an averaged diameter in the range of 3 to 50 µm, more preferred in the range of 5 to 40 µm and mostly preferred from 5 to 20 µm. In case the particle size is above the upper limit the pressure stability of the sorbent and the linear flow rate during chromatographic separation is decreased. The determination of the particle size is made via laser diffraction according to ISO 13320 (2009).

The pores of the porous particles preferably have an averaged diameter in the range of 3 to 50 nm. The pore diameter is determined via inverse size exclusion chromatography: thereby, the phase material to be determined is packed in a chromatographic column and a series of size standard polymers are injected. The curve progression of a diagram of the logarithm of the mol-mass of the respective standard against the elution volume leads to the pore diameter distribution and the averaged pore diameter.

The pore volume of the porous particles is preferably in the range of 1 to 3 ml/g. The pore volume is determined via measurement of the water absorption capacity: the solvent for which the pore volume is to be determined (different solvents may show different results due to different wettability) is added to the phase material, the weight of which is determined in the dry state. For the purpose of the present invention water is preferably used as the solvent. Excessive solvent is filtered off and the phase material is freed via centrifugation from solvent in the void volume. Subsequently, the material is weighed again. In this case only the pores should be filled with the solvent. Via mass difference between the material with filled and empty pores as well as the density of the solvent the pore volume can be calculated.

The production of the crosslinked sulfonated aromatic polymer is preferably made via sulfonation of an already crosslinked aromatic polymer by adding sulphuric acid or similar materials, as is for example known by the production of sulfonated crosslinked polystyrene from GB 1116800 and GB 1483587. The production of crosslinked polymers is state of the art and can be made by a person skilled in the field of polymer chemistry without inventive effort. It is particularly preferred that the sulfonation is made as follows: Depending on the desired degree of sulfonation for instance a polystyrene-divinylbenzol-polymer is stirred in a mixture of sulphuric acid and water having a volume percentage of water of 2 to 15 % at temperatures in the range of 20°C to 80°C for 1 to 6 hours. Increasing the amount of sulphuric acid, of the temperature or the reaction time leads to an enhancement of the degree of sulfonation. By adjusting all of these three parameters the desired degree of sulfonation can be achieved in a very exact manner. After the reaction, the polymer is preferably washed with diluted sulphuric acid (62 %), water and methanol.

The porous particles of the crosslinked sulfonated aromatic polymer preferably have a cation exchange capacity in the range of 200 to 800 µmol/ml. The cation exchange capacity is measured by loading a column with a defined volume with an excess of 100 mM ammonium acetate solution. The ammonium is then eluted with a well-defined volume of 100 mM sodium acetate solution. Then the concentration of ammonium is determined by using Ammonia vial test (Hach TNT832) and the cation exchange capacity of the column is calculated. The advantage of a cation exchange capacity in the above-range is that the hydrophilic polymer can be easy applied to the porous particles, since the hydrophilic interaction is sufficiently high.

The hydrophilic polymer which contains a ligand group in the side chain (derivatized hydrophilic polymer) is preferably produced in that a hydrophilic polymer (non-derivatized hydrophilic polymer) is reacted with a derivatizing reagent. Thereby the functional group of the non-derivatized hydrophilic polymer is reacted with the derivatizing reagent by forming a covalent bond.

The non-derivatized hydrophilic polymer is preferably one wherein a hydrophilic group is in the main chain or the side chain of the polymer. A hydrophilic group in the side chain is however further preferred.

Preferred hydrophilic groups are -NH₂, -NH-, -OH, -COOH, - OOCCH₃, anhydrides, -NHC(O)- and saccharides, wherein -NH₂ and -OH are further preferred, and -NH₂ is most preferred, i.e. an amine group-containing polymer. Examples of such polymers are: polyamines, i.e. polyvinyl amine, polyethylene amine, polyallyl amine, polyamine acids, such as polylysine etc., polyvinyl alcohol, polyvinyl acetate, polyacrylic acid, polymethacrylic acid, polyamides and polysaccharides (cellulose, dextran, pullulane etc.), wherein polyamine, such as i.e. polyvinyl amine and polyallyl amine are further preferred and polyvinyl amine is most preferred.

The derivatized hydrophilic polymer - as is present as a coating on the porous particles - is preferably one of the before-mentioned non-derivatized hydrophilic polymers, which is derivatized by a ligand group being an aliphatic hydrocarbon group having 3 to 20 carbon atoms.

The derivatized hydrophilic polymer comprises or consists of individual chains which are preferably covalently crosslinked with each other. Furthermore, the polymer is preferably not covalently bound to the surface of the solid support material. The inventors of the present invention have surprisingly found out that especially for the purification/separation of insulin having both a hydrophobic and a hydrophilic moiety it is important that the polymer is flexible enough to come into a conformation which makes it possible that both the hydrophobic and the hydrophilic (e.g. hydrogen donor or acceptor interactions) moieties may come into contact with hydrophobic and hydrophilic moieties of the compound to be purified. In case a polymer film would be used which is covalently bound to the surface of the support material the inventors of the present invention observed that the purification capacity significantly decreased. That is, the use of a non-covalent surface bound crosslinked polymer as a polymer film has three advantages: (1) flexibility of the polymer due to the fact that it is not surface bound; (2) the crosslinking ensures that the film is adhered to the surface of the porous particles (support material) and is not lost; (3) the thickness of the hydrophilic polymer can be adjusted as thin as wanted, if the polymer is not covalently bound to the porous particles.

The crosslinking degree of the hydrophilic polymer is at least 2 %, based on the total number of crosslinkable groups in the hydrophilic polymer. More preferred the crosslinking degree is of from 3 to 50 %, more preferred of from 4 to 30 %, most preferred from 5 to 15 %, based on the total number of crosslinkable groups in the hydrophilic polymer. The crosslinking degree can easily be adjusted by the stoichiometric amount of the crosslinking reagent used. It is assumed that nearly 100 mol% of the crosslinking reagent reacts and forms crosslinks. This can be verified by analytical methods. The crosslinking degree can be determined by MAS-NMR spectroscopy and quantitative determination of the amount of crosslinker in relation to the amount of polymer. This method is most preferred. The crosslinking degree can also be determined by IR spectroscopy based on e.g. C-O-C or OH vibrations using a calibration curve. Both methods are standard analytical methods for a person skilled in the art. If the crosslinking degree is above the upper limit the polymer film suffers in flexibility thereby resulting in an inferior purification capacity. If the crosslinking degree is below the limit mentioned above the film suffers in stability on the surface of the porous particles.

The crosslinking reagent used for crosslinking the hydrophilic polymer is preferably selected from the group consisting of dicarboxylic acids, diamines, diols, urea and bis-epoxides, more preferred dicarboxylic acids and bis-epoxides, such as terephthalic acid, biphenyl dicarboxylic acid, 1,12-bis-(5-norbornen-2,3-dicarboximido)-decandicarboxylic acid and ethylene glycol diglycidylether, ethylene glycol diglycidylether being most preferred. In one embodiment the at least one crosslinking reagent is a linear, conformationally flexible molecule of a length of between 4 and 20 atoms.

The ratio of the weight of the hydrophilic polymer coating the porous particles to the weight of the porous particles preferably ranges from 0.01 to 0.2, more preferably 0.05 to 0.15, in the sorbent according to the invention. If the above ratio is above the upper limit, the polymer film tends to be thicker and the pores of the porous particles are mainly covered resulting in a sorbent having no available pores. If the above ratio is below the lower limit, the amount of polymer is too low to cover the surface of the porous particles sufficiently. Furthermore, in the latter case more crosslinking reagent is necessary in order to fix the hydrophilic polymer to the porous particles, again resulting in a hydrophilic polymer film suffering in flexibility.

The hydrophilic polymer can be applied to the porous particles by all means of coating known to a person skilled in the art such as absorption, vapor phase deposition, polymerization from the liquid, gas or plasma phase, spin coating, surface condensation, wetting, soaking, dipping, rushing, spraying, damping, evaporation, incipient wetness, application of electric fields or pressure, as well as methods based on molecular self-assembly such as, for example, liquid crystals, Langmuir Blodgett- or layer-by-layer film formation. The polymer may thereby be coated directly as a monolayer or as multilayer or as a stepwise sequence of individual monolayers on top of each other. It is preferred in the present invention that the hydrophilic polymer is coated to the porous particles by incipient wetness. The crosslinking of the hydrophilic polymer is made after it has been applied to the porous particles.

As pointed out above, the non-derivatized hydrophilic polymer preferably contains functional groups which are derivatized by the above-mentioned ligand group. For this reason a derivatizing reagent is used which contains the ligand group. With respect to a superior purification capacity it is preferred that in the sorbent according to the invention the molar ratio of the ligand groups to the amount of functional groups of the non-derivatized hydrophilic polymer (derivatization degree) is preferably in the range of 0.1 to 0.5, more preferred in the range of 0.2 to 0.4, wherein the amount of functional groups is determined by titration of the sorbent before the ligand groups have been applied and after the non-derivatized hydrophilic polymer has been crosslinked, and wherein the amount of ligand groups is determined in that the amount of functional groups have been determined in the same way, but after the derivatization, and the latter amount is substracted from the former amount.

The term *"functional group"* means any simple, distinct chemical moiety belonging to the hydrophilic polymer. Thereby, the functional group may serve as chemical attachment point or anchor. Functional groups preferably contain at least one weak bond and/or one heteroatom, preferably a group behaving as nucleophil or electrophil. The preferred functional groups are primary and secondary amino, hydroxyl, and carboxylic acid or ester groups, when taken before the derivatizing reagents have been bound to these groups. When the derivatizing reagents are bound to the functional groups the nature of these groups change with respect to the structure of the ligand group.

The ligand group of the sorbent according to the invention comprises an aliphatic hydrocarbon group having 3 to 20 carbon atoms, preferably 3 to 15 carbon atoms, further preferred 4 to 10 carbon atoms and most preferred 4 to 8 carbon atoms.

The aliphatic hydrocarbon group may be a linear, branched or cyclic hydrocarbon group, wherein linear and branched hydrocarbon groups are particularly preferred, and a branched hydrocarbon group is most preferred.

Examples of the aliphatic hydrocarbon groups are the following: methyl, ethyl, n-propyl, iso-propyl, n-butyl, isobutyl, sec-butyl (1-methylpropyl), tert-butyl, iso-pentyl, n-pentyl, tert-pentyl (1,1- dimethylpropyl), 1,2-dimethylpropyl, 2,2-dimethylpropyl (neopentyl), 1-ethylpropyl, 2-methylbutyl, n-hexyl, iso-hexyl, 1,2-dimethylbutyl, 1-ethyl-1-methylpropyl, 1-ethyl-2-methylpropyl, 1,1,2-trimethylpropyl, 1,2,2-trimethylpropyl, 1-ethylbutyl, 1-methylbutyl, 1,1-dimethylbutyl, 2,2-dimethylbutyl, 1,3-dimethylbutyl, 2,3-dimethylbutyl, 3,3-dimethylbutyl, 2-ethylbutyl, 1-methylpentyl, 2-methylpentyl, 3-methylpentyl, 4-methylpentyl, n-heptyl, n-octyl, n-nonyl, n-decyl, n-undecyl, n-dodecyl, n-tridecyl, n-tetradecyl, n-pentadecyl, 1-hexylnonyl, n-hexadecyl, 1-hexyl-decyl, n-heptadecyl, n-octadecyl, n-nonadecyl, -(CH₂)₂₀CH₃, -(CH₂)₂₁CH₃, -(CH₂)₂₂CH₃, cyclopentyl, cyclohexyl, cycloheptyl, cyclooctyl, 2-ethylhexyl, trifluormethyl, pentafluorethyl, 2,2,2-trifluorethyl, ethenyl, propenyl, butenyl, pentenyl, cyclopentenyl, hexenyl, cyclohexenyl, heptenyl, cycloheptenyl, octenyl or cyclooctenyl, wherein the representatives having 6 carbon atoms are preferred, and 4-methylpentyl being most preferred.

Before being bound to the hydrophilic polymer, the ligand group is part of a derivatizing reagent, the derivatizing reagent further comprising a coupling group which is able to react with the functional group of the hydrophilic polymer.

The present invention also relates to a method for preparing a sorbent (production method), preferably the sorbent according to the invention, comprising:
(i) providing porous particles of a crosslinked sulfonated aromatic polymer;
(ii) coating the porous particles with a hydrophilic polymer;
(iii) crosslinking the hydrophilic polymer; and
(iv) binding a derivatizing reagent to the hydrophilic polymer, wherein the derivatizing reagent comprises an aliphatic hydrocarbon group having 3 to 20 carbon atoms (having the same definition as above).

All embodiments with respect to the production of the sorbent according to the invention mentioned above should be read as embodiments of the method of producing according to the invention, and vice versa. In the same way all embodiments mentioned above in connection with the sorbent according to the invention are valid for the sorbent manufactured in the method of producing according to the invention.

For the coating of the porous particles with a hydrophilic polymer, the hydrophilic polymer is preferably solved in an aqueous solvent wherein the pH is suitably adjusted in order to solve or suspend the polymer. Preferably the pH of the aqueous solvent is adjusted to be in the range of 8 to 11, further preferred in the range of 8.5 to 10. A mineral acid is preferably used for the adjustment of the pH of the solution in the above pH-range, in that it is added to the aqueous solution containing the hydrophilic polymer. As mineral acid hydrochloric acid is preferred, in particular concentrated hydrochloric acid.

The coating of the porous particles is preferably done by bringing into contact the aqueous solution containing the hydrophilic polymer with the particles. It is preferred that the coating is made by using the so-called *"incipient* wetness"-method. By this method a volume of the aqueous solution is taken which correlates to the pore volume of the particles. It is preferred that the particles and the aqueous solution are put in a drum, preferably on a sieving machine, wherein the resulting mixture is blended for preferably up to six hours. Or the particles and the aqueous solution of the polymer are mixed in the drum of a Plough-share mixer for 2 h. During this mixing the hydrophilic polymer is taken up by the pores of the porous particles. The amount of hydrophilic polymer in the aqueous solution is preferably chosen such that the ratio of the weight of the hydrophilic polymer to the weight of the porous particles ranges from 0.01 to 0.2, more preferably 0.05 to 0.15. That is, the concentration of the hydrophilic polymer in the aqueous solution results from the before-mentioned amount and the fact that the amount of solvent is dependent on the volume of the solution which should correlate to the pore volume of the porous particles.

The coated particles are then dried, preferably under reduced pressure, preferably under elevated temperatures such as in the range of 40 to 60°C. The drying may occur in a vacuum drying oven or in a heated ploughshare mixer equipped with a drying tower under reduced pressure

For the crosslinking step, the coated particles are preferably suspended in an organic solvent, such as isopropanol or dimethylformamide (DMF), more preferred isopropanol. This step may be conducted in a stirred batch reactor. The crosslinking reagent is then added to the suspension, preferably in an amount of 3 to 50 %, more preferred of 4 to 30 %, most preferred of 5 to 15 %, based on the total number of crosslinkable groups in the hydrophilic polymer. The mixture is then preferably heated up to a temperature between 40 and 70°C, for preferably 4 to 8 hours. After the reaction the suspension is preferably washed, preferably on a suction strainer. Suitable washing solvents are diluted hydrochloric acid, water and NaOH-solution. Most preferred is that the resulting particles are firstly washed with diluted hydrochloric acid, then water, then NaOH-solution and again water. Subsequently, the porous particles coated with the crosslinked hydrophilic polymer are preferably dried, preferably under reduced pressure, preferably in a temperature range of 40 to 60°C. The drying may be conducted in a vacuum drying oven.

Depending on the kind of functional groups of the hydrophilic polymer and depending on the coupling group of the derivatizing reagent different strategies for the binding of the derivatizing reagent to the hydrophilic polymer may be used. If the hydrophilic polymer contains amine groups as functional groups, radicals containing a carboxylic acid group can be attached to the amine nitrogen atom via the carboxylic carbon atom via peptide chemistry. For this reaction coupling reagents such as 2-(1H-benzotriazole-1-yl)-1,1,3,3-tetramethyluronium hexafluorophosphate (HBTU), O-(1H-6-chlorobenzotriazole-1-yl)-1,1,3,3-tetramethyluronium hexafluorophosphate (HCTU), benzotriazole-1-yl-oxy-tris-pyrrolidino-phosphonium hexafluorophosphate (PyBOP), propylphosphonic anhydride (T3P) etc. or reactive groups in the reagent like isocyanates, epoxides on anhydrides may be used. If the hydrophilic polymer contains amine groups, aliphatic carbon atoms of the derivatizing reagent may be bound to the amine nitrogen atom via a nucleophilic aliphatic substitution.

If the hydrophilic polymer contains hydroxyl groups, a derivatizing reagent containing a carboxylic acid chloride may be reacted with the hydroxyl group by forming an ester bond.

The person skilled in the field of organic synthesis knows possible reactions of functional groups of the hydrophilic polymer with derivatizing reagents in order to obtain the sorbents according to the invention.

For the binding of the derivatizing reagent to the hydrophilic polymer, the coated particles are preferably suspended in an organic solvent, such as DMF. The resulting suspension is preferably heated up to a temperature of 40 to 60°C, preferably for 1 to 3 hours. During the heating the suspension is preferably stirred, preferably in a stirred batch reactor. The derivatizing reagent is subsequently added to the suspension, if necessary, additionally activation reagent. In case of a peptide bonding the activation reagent may be a mixture of HBTU and a base such as triethylamine.

The advantages of the present invention are as follows:
- The main advantage is the combination of different selective principles based on the bimodal or multimodal phase design of the sorbent according to the invention: The combination of anion-exchange binding mode with the lipophilic groups of the ligand groups leads to an extraordinary high selectivity and load capacity compared to usual C18 phases.
- The selectivity between Insulin and its most prominent by-product, the A21-desamido-Inslulin is larger than 5, compared with ∼1.5 on the most widely used C18 phases.
- Additionally, the use of porous particles of a crosslinked sulfonated aromatic polymer as well as the high stability of the coating allows the simple sanitization with cheap

sodium hydroxide. This procedure is not possible on silica-based C18-phases, widely used for Insulin polishing.
- On the other hand, simple ion exchanger does not provide such a high selectivity, as the sorbent according to the invention does, because of the combination of different binding principles - the bimodal or multimodal approach.

The present invention is further explained by means of the following figures and examples which should however not be understood as being limiting for the scope of the present invention:

### Figures:

- Fig. 1:: Sample curve for the determination of the amount of amine groups by means of break-through measurement with 4-toluene sulfonic acid (front analysis).
- Fig. 2:: Separation of insulin from its critical impurity desamidoinsulin by using a sorbent according to the invention (Example 1).
- Fig. 3:: Separation of insulin from its critical impurity desamidoinsulin with variation of the n-propyl alcohol content.
- Fig. 4:: Separation of insulin from its critical impurity desamidoinsulin by using the sorbent according to the invention (Example 1).
- Fig. 5:: Separation according to Example 5.
- Fig. 6:: Analytical chromatogram of the fractions collected during the separation shown in Figure 5 (Example 5).
- Fig. 7:: Chromatographic profiles of analytical injections before (top) and after (down) 1005 sanitisation cycles by using the sorbent according to Example 1 for the purification of insulin.

### Examples:

### Analytical methods:

Determination of the amount of amine groups by means of break-through measurement with 4-toluene sulfonic acid (front analysis) (titration method):
The respective sorbent is packed to a column having the dimensions 33.5 x 4 mm (bed volume 0.42 mL). The filled column is then flushed with the following media at a flow rate of 1.0 mL/min:
   - 5 mL of water
   - 10 mL of a 100 mM aqueous solution of ammonium acetate
   - 1 mL of water
   - 10 mL of a 100 mM aqueous solution of trifluoroacetic acid
   - 10 mL of water

A base line is detected at a HPLC-device having a pump and a UV-detector after water has been pumped through the device for 5 min at 0.5 mL/min. After that a solution of 10 mM 4-toluene sulfonic acid in water is pumped through, whereas the extinction of the eluent is detected at 274 nm. The extinction rises in few minutes to a level of about 700 mAU and remains constant at this level (flush-in curve). After 25 min the column is applied between pump and detector and is flushed with 10 mM of 4-toluene sulfonic acid at 0.5 mL/min. The extinction then drops to 0 mAU since the column is binding 4-toluene sulfonic acid. If the capacity of the column is exhausted, the extinction of the eluate again rises to the starting level of ∼700 mAU.

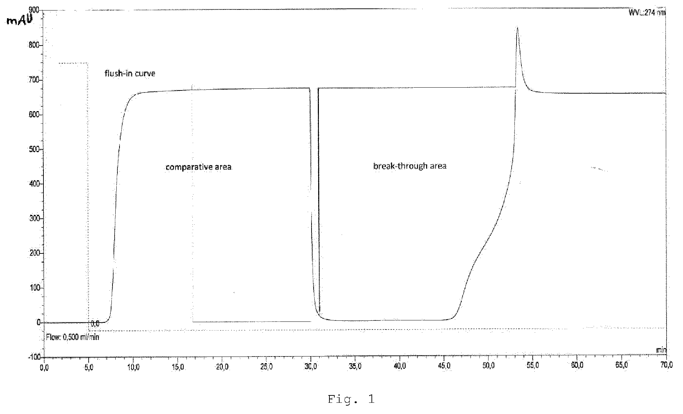

For the determination of the capacity of 4-toluene sulfonic acid the area below the level of the flush-in curve is integrated as comparative area, thereby obtaining the relationship between surface area and the amount of 4-toluene sulfonic acid. After that the area (break-through area) of the toluene sulfonic acid solution absorbed by the column is titrated, and the volume of the device and the dead volume of the column (0.5 mL) are subtracted. The break-through area directly indicates the amount of 4-toluene sulfonic acid bound to the column. Dividing this amount by the volume of the column yields in the capacity of toluene sulfonic acid per mL of the sorbent, also resulting in the amount of amine groups of the sorbent. For the better understanding of this method Fig. 1 shows such an example curve.

Example 1: Method of producing a sorbent according to the invention:
Step 1: Sulfonation:
   100 g of TreverChrom CHR P 10/300 was added into to a stirred 1 L batch reactor filled with 950 g of sulfuric acid (98%). The suspension was heated to 40 °C for 4 h. Then the sulfuric acid was filtered of on a suction strainer. The sulfonated particles were washed with the following solvents: 1 L diluted sulfuric acid (62%), 1 L water, 1 L methanol, 1 L water and 1 L methanol. The stationary phase was dried then under reduced pressure (2 mBar) in a vaccum oven to constant weight.
      Yield: 114.2 g
      Cation exchange capacity: 493 µmol/mL
      Pore volume: 0.83 mL/g
Step 2: Coating:
   10 g of the sulfonated polystyrene particles are weighed in a 100 mL polyethylene flask. To 6.45 g of a polyvinylamine solution (polymer content 12.4 %) hydrochloric acid was added until the pH is 9.5 (0.44 g HCl (32%)). The polyvinylamine solution was diluted by adding 1.4 g of water. This polyvinylamine solution was filled to 10 g of the sulfonated polystyrene particles weight in a 100 mL polyethylene flask and mixed for 6 hours on a sieving machine. Then the coated particles were dried to constant under reduced pressure in a vaccum oven at 50 °C.
      Yield: 10.54 g
Step 3: Crosslinking:
   10.54 g of the coated polymer particles were suspended in 100 mL isopropanol in a 250 mL stirred batch reactor. Then 0.488 g of the crosslinker ethylene glycol diglycidyl ether [2224-15-9] was added and the suspension was heated to 55°C for 6 h. The stationary phase was filtered on a suction strainer and washed with the following solvents: 120 mL isopropanol, 240 mL 0,1 M hydrochloric acid, 120 mL water, 120 mL 1 M sodium hydroxide, 120 mL water and 120 mL methanol.
      Then the coated particles were dried to constant under reduced pressure in a vaccum oven at 50 °C.
      Yield: 9.9 g
      Anion exchange capacity (TSS): 300 µmol/mL
Step 4: Derivatisation:
   8 g of the crosslinked stationary phase is suspended in 50 mL dimethylformamide and stirred in a 250 mL batch reactor at 50°C. After addition of 277 µL 4-methylpentanoic acid [646-07-1] as ligand, 820 mg 2-(1H-benzotriazol-1-yl)-1,1,3,3-tetramethyluronium hexafluorophosphate [94790-37-1] as coupling reagent and 306 µL trimethylamine [121-44-8] as base to the suspension, the reaction temperature was kept for 2 h. Then the stationary phase was filtered on a suction strainer and washed with the following solvents: 120 mL dimethylformamide, 120 mL acetic acid, 120 mL water, 120 mL 1 M sodium hydroxide, 120 mL water and 120 mL methanol.Then the coated particles were dried to constant under reduced pressure in a vaccum oven at 50 °C.
      Yield: 8.65 g
      Anion exchange capacity (TSS): 225 µmol/mL

### Example 2: Analytical separation of an insulin sample containing 15 wt.-% Desamidoinsulin ("A21") and 85 wt.-% insulin.

For the separation human insulin is used having a concentration of 1 mg/mL in H₂O/MeCN/TFA (95/5/0,1). The sorbent according to Example 1 is used as the column material. The analytical separation is made via a HPLC system from Dionex (former Gynkotek) which consist of a four channel low-pressure gradient pump (LPG 580, LPG 680 or LPG 3400), auto sampler (Gina 50, ASI-100 or WPS-300), six-channel column switching valves (Besta), column oven and diode-array uv detector (UVD 170U, UVD 340S or VWD 3400). Further conditions: Column Size: 40 x 4 mm, flow rate: 1 ml/min, detection: UV at 214 nm, injection volume 20 µl, column temperature 25°C.

The gradient used for the separation of the insulin sample is shown in Table 1.

**Table 1:**

| time [min] | buffer A | buffer B | n-propyl alcohol |
|---|---|---|---|
| 0 | 60 | 10 | 30 |
| 45 | 60 | 10 | 30 |
| 45 | 20 | 50 | 30 |
| 50 | 20 | 50 | 30 |
| 50 | 60 | 10 | 30 |
| 60 | 60 | 10 | 30 |

| | | | |
|---|---|---|---|
| Buffer A: 10 mM NaK-phosphate buffer, pH 7.3; buffer B: 10 mM NaK-Phosphate buffer, 1M NaCI, pH 7.3. | | | |

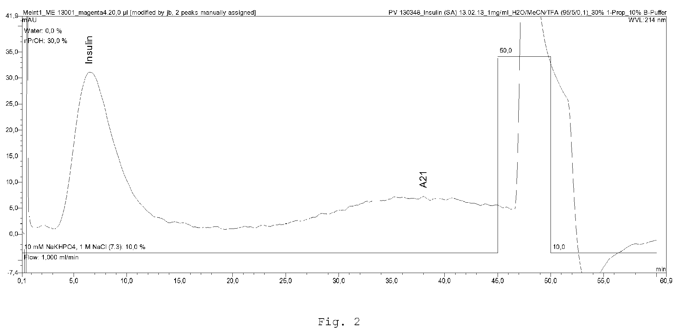

The separation of insulin from its critical impurity desamidoinsulin is shown in Figure 2. The obtained α-value (insulin/desamidoinsulin) is >6.

### Example 3: Analytical separation according to Example 2 (changes mentioned), wherein the n-propyl alcohol content is varied and its influence on k'-value is determined:

The gradient used for the separation of the insulin sample is shown in Table 2.

**Table 2:**

| time [min] | buffer A | buffer B | n-propyl alcohol |
|---|---|---|---|
| 0 | 90-X | 10 | X |
| 45 | 90-X | 10 | X |
| 45 | 50-X | 50 | X |
| 50 | 50-X | 50 | X |
| 50 | 90-X | 10 | X |
| 60 | 90-X | 10 | X |

| | | | |
|---|---|---|---|
| X: 25-35%, buffer A: 10 mM NaK-phosphate buffer, pH 7.3; buffer B: 10 mM NaK-phosphate buffer, 1M NaCl, pH 7.3. | | | |

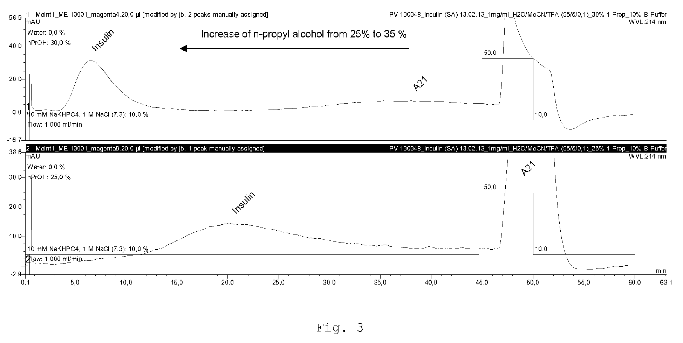

The influence of the n-propyl alcohol content on the k'-value is shown in Table 3 and Figure 3 (upper graph 30 % n-propyl alcohol, lower graph 25 % n-propyl alcohol).

**Table 3:**

| n-propyl alcohol [%] | k'-value (insulin) |
|---|---|
| 25 | 20 |
| 30 | 68 |

The analytical separation is made by the following conditions:
Column Size: 40 x 4 mm, flow rate: 1 ml/min, detection: UV at 214 nm, injection volume 20 µl, column temperature 25°C, sample load (insulin, 1 mg/mL in H₂O/MeCN/TFA (95/5/0,1)),
   gradient: see Table 2.

### Example 4: Analytical separation according to Example 2 (changes mentioned):

The insulin sample used is human insulin in a concentration of 35 mg/mL in 20 mM HCl. The insulin sample contains 15 wt.-% Desamidoinsulin ("A21") and 85 wt.-% insulin.

The gradient used for the separation of the insulin sample is shown in Table 4.

**Table 4:**

| time [min] | buffer A | buffer B | n-propyl alcohol |
|---|---|---|---|
| 0 | 65 | 10 | 25 |
| 100 | 65 | 10 | 25 |
| 100 | 20 | 50 | 30 |
| 120 | 20 | 50 | 30 |
| 120 | 65 | 10 | 25 |
| 140 | 65 | 10 | 25 |

| | | | |
|---|---|---|---|
| Buffer A: 10 mM NaK-phosphate buffer, pH 7.3; buffer B: 10 mM NaK-phosphate buffer, 1M NaCl, pH 7.3. | | | |

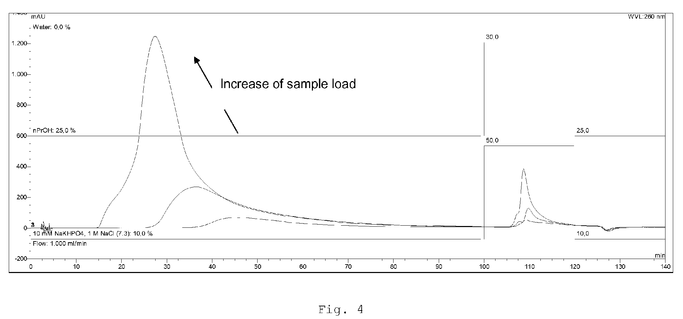

The separation of insulin from its critical impurity desamidoinsulin is shown in Figure 4.

The analytical separation is made by the following conditions:
Sample load increase from 0.22% (w/w, Rₜ (Insulin) ∼ 45 min) to 2.72% (w/w, Rₜ (Insulin) ∼ 28 min); Column Size: 250 x 4 mm, flow rate: 1, detection: UV at 260 nm, injection volume 85 µl - 1070 µl, column temperature 25°C, sample load (insulin, c = 35 mg/ml in 20 mM HCl), gradient: see Table 4.

### Example 5: Purification of an insulin sample with starting purity of 87 w.-% of insulin (impurity desamidoinsulin 13 wt.- %) to a final purity of > 99%.

The purification was performed using the same conditions as described in Example 4.

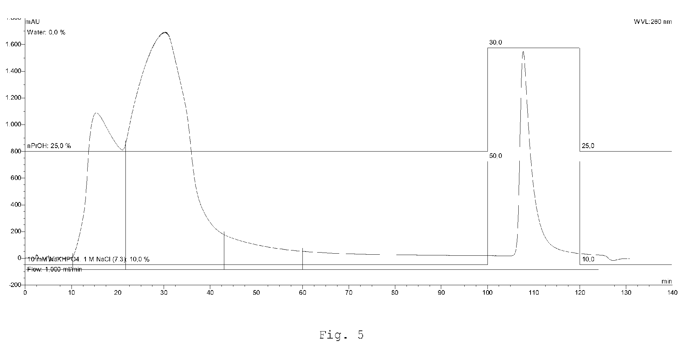

Figure 5 shows the separation according to Example 5. The conditions were as follows: Fraction collection (sample load 6% (w/w); Column Size: 250 x 4 mm, flow rate 1 ml/min, detection: UV at 260 nm, injection volume 85-1070 µl, column temperature 25°C, sample load (Insulin, c = 35 mg/ml in 20 mM HCI): 0.22; 0.86; 2.72 % (w/w), gradient: see table 3.

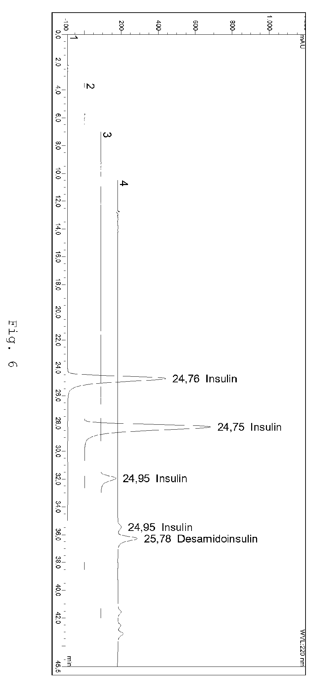

Figure 6 shows the analytical chromatogram of the fractions collected during the separation shown in Figure 5. The desamidoinsulin is only detectable in the last fraction (chromatogram 4) . Chromatogram 1: Fraction 02, 10 - 21.5 min; Chromatogram 2: Fraction 03, 21.5 - 43 min; Chromatogram 3: Fraction 04, 43 - 60 min; Chromatogram 4: Fraction 06, 104-124 min; conditions: column Size: 250 x 4 mm, flow rate: 1 ml/min, detection: UV at 220 nm, injection volume 20 µl, column temperature 25°C, gradient: 0-18 min: increase buffer B from 45% to 50%; 18-28 min: increase buffer B from 50% to 65% buffer B, 28-30 min: 65% buffer B; 30-31 min: decrease buffer B from 65% to 45%; 31-35 min: 45% buffer B.
Buffer A: 140 mM NaClO₄, 34 mM TEA, 60 mM NaH₂PO₄ (pH 2.3), 5.5% ACN (w/w), buffer B: 70 mM NaCI0₄, 17 mM TEA, 110 mM NaH₂PO₄ (pH 2.3), 50% ACN (w/w).

### Example 6: Stability against 1M sodium hydroxide

To monitor the stability against 1M sodium hydroxide (sanitization agent), 1005 sanitization cycles (each cycle: 15 min 1M Na0H, static or in flow) were performed and chromatographic profiles of analytical insulin injections compared.

The stability test against 1 M sodium hydroxide was carried out in the following way:
- 100 load/ CIP cycles real (each 1M NaOH 30 min in flow)
- 400 CIP simulated (400 x 30 min 1M NaOH static)
- 100 load/CIP cycles real
- 400 CIP cycles simulated
- 5 load/CIP cycles real

"Real load cycles" means real injections of Insulin and monitoring of retention and selectivity against "A21". The simulated CIP-cycles consist of sodium hydroxide flushing, incubation and reconditioning to separation conditions without injection of Insulin. This reduced cycle was repeated as shown above. The integrity of the stationary phase was monitored by analytical injections as described below.

The gradient used for the separation of insulin is shown in table 5:

**Table 5:**

| time [min] | buffer A | buffer B | n-propyl alcohol |
|---|---|---|---|
| 0 | 55 | 10 | 25 |
| 45 | 55 | 10 | 25 |
| 45 | 25 | 50 | 25 |
| 50 | 25 | 50 | 25 |
| 50 | 55 | 10 | 25 |
| 60 | 55 | 10 | 25 |

| | | | |
|---|---|---|---|
| Buffer A: 10 mM NaK-phosphate buffer, pH 7.3; buffer B: 10 mM NaK-Phosphate buffer, 1M NaCl, pH 7.3. | | | |

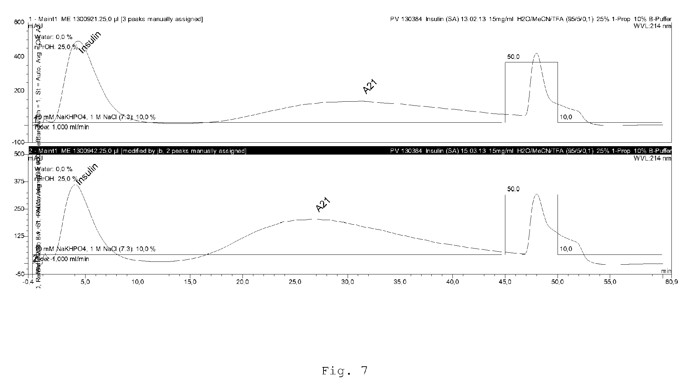

Figure 7 shows chromatographic profiles of analytical injections before (top) and after (down) 1005 sanitisation cycles. This means that the stability of the sorbent according to Example 1 against 1M NaOH as sanitisation reagent is very high.

Conditions: Column Size: 250 x 4 mm, flow rate: 1 ml/min, detection: UV at 220 nm, injection volume 85 µl, column temperature 25°C, sample load (Insulin, c = 35 mg/ml in 20 mM HCI): 0.22 % (w/w), gradient: See table 5.

## Claims

1. Use of a sorbent for the separation of insulin or a derivative of insulin from a solution, wherein the sorbent comprises porous particles of a crosslinked sulfonated aromatic polymer, the porous particles being coated with a hydrophilic polymer, wherein the hydrophilic polymer comprises in its side chain a ligand group, the ligand group being an aliphatic hydrocarbon unit with 3 to 20 carbon atoms.

2. Use according to claim 1, wherein the crosslinked sulfonated aromatic polymer comprises a polyvinyl skeletal structure.

3. Use according to claim 1 or 2, wherein the crosslinked sulfonated aromatic polymer comprises a phenyl sulfonic acid group in its side chain.

4. Use according to any one of claims 1 to 3, wherein the crosslinked sulfonated aromatic polymer is a sulfonated copolymer of polystyrene and divinylbenzol.

5. Use according to any one of claims 1 to 4, wherein the particles have an averaged diameter in the range of 3 to 50 µm.

6. Use according to any one of claims 1 to 5, wherein the particles have pores with an averaged diameter in the range of 3 to 50 nm.

7. Use according to any one of claims 1 to 6, wherein the hydrophilic polymer is a crosslinked polymer.

8. Use according to any one of claims 1 to 7, wherein the hydrophilic polymer is an amine-group containing polymer.

9. Use according to any one of claims 1 to 8, wherein the ligand group is an aliphatic hydrocarbon unit having 3 to 10 carbon atoms.

10. Use according to any one of claims 1 to 9, wherein the ligand group is 4-methyl-pentyl.

11. Sorbent comprising porous particles of a crosslinked sulfonated aromatic polymer being coated with a hydrophilic polymer, wherein the hydrophilic polymer comprises a ligand group in its side chain, the ligand group being an aliphatic hydrocarbon unit having 3 to 20 carbon atoms.

12. Sorbent according to claim 11, wherein the hydrophilic polymer is a polyvinyl amine.

13. Sorbent according to claim 11 or 12, wherein the crosslinked sulfonated aromatic polymer is a sulfonated copolymer of polystyrene and divinylbenzol.

14. Sorbent according to any one of claims 11 to 13, wherein the ligand group is an aliphatic hydrocarbon unit having 4 to 8 carbon atoms.

15. Method for preparing a sorbent comprising the following steps in the sequence as stated below:
(a) providing porous particles of a crosslinked sulfonated aromatic polymer;
(b) coating of the porous particles with a hydrophilic polymer;
(c) crosslinking of the hydrophilic polymer; and
(d) binding a derivatizing reagent to the hydrophilic polymer, wherein the derivatizing reagent comprises an aliphatic hydrocarbon group having 3 to 20 carbon atoms.
